# EUROPEAN PATENT SPECIFICATION

(11) **EP 1 526 849 B1**
(45) Date of publication and mention of the grant of the patent: **05.04.2006**
(21) Application number: 03766339.0
(22) Date of filing: 29.07.2003
(51) Int. Cl.: A61K 31/19, A61P 29/00

(54) **TOPICAL FORMULATIONS CONTAINING KETOPROFEN STABILISED WITH SULISOBENZONE**
TOPISCHE ZUSAMMENSETZUNG ENTHALTEND KETOPROFEN STABILISIERT MIT SULISOBENZON
FORMULATIONS TOPIQUES CONTENANT DU KETOPROFENE STABILISEES AU SULISOBENZONE

(30) Priority: 01.08.2002 IT FI20020144
(43) Date of publication of application: 04.05.2005
(73) Proprietor: A. MENARINI INDUSTRIE FARMACEUTICHE RIUNITE S.R.L., I-50131 Firenze (IT)
(72) Inventor: BACCANI CARIDI, Claudio, IT-50065 Pontassieve (IT); TOSETTI, Alessandro, I-50132 Bagno A Ripoli (IT)
(74) Representative: Gervasi, Gemma
(86) International application number: PCT/EP2003/008351
(87) International publication number: WO 2004/012725

(56) References cited:
- DATABASE WPI Derwent Publications Ltd., London, GB; AN 1985-239345 XP002264779 NAKAGAWA ET AL : "Stable pharmaceutical for external use containing ketoprofen" & JP 60 155111 A (HISAMITSU), 15 August 1995 (1995-08-15)
- DATABASE WPI Derwent Publications Ltd., London, GB; AN 2000-551547 XP002264780 YANAGIBASHI: "Antiohlogistic sedative drug for external use" & JP 2000 178192 A (LION CORP)
- LE COZ ET AL: "Photocontact dermatitis from ketoprofen and tiaprofenic acid. " CONTACT DERMATITIS, vol. 38, 1998, pages 245-252, XP009022959

## Description

### Field of the invention

The object of the present invention are formulations containing ketoprofen or its S(+) isomer or mixtures of the two isomers or pharmaceutical acceptable salts thereof, together with a UV filter, sulisobenzone, and an antioxidant, butyl hydroxy anisole, in concentrations greater than 0.05% and up to 0.2%.

### State of the art

Topical pharmaceutical formulations containing non steroidal anti-inflammatory, agents (NSAIs), in particular ketoprofen and dexketoprofen, are described in the majority of the scientific literature as irritants, allergens, phototoxins and photo-allergenics (Coz, Christophe J. *et al* Contact Dermatitis, 38 (5), 245ff (1989), Bosca, F. J. Photochem. Photobiol., 43(3), 1ff (1998)).

A number of studies have been conducted in the past on the photochemical stabilisation of formulations containing NSAls. It has been in fact attempted to insert NSAls, in particular ketoprofen or dexketoprofen, into ointment creams or gels in a way such as to obtain stable formulations, both physically and chemically. The works of Coz, Christophe J and Bosca F. could demonstrate that some parts of the NSAI structure were inert whilst others were photosensitive. In the case of ketoprofen it is the benzophenone part which is implied in the photo-degradation whilst the propionic acid residue is practically insensitive to interaction with light. The specific photosensitivity of the diphenyl ketonic group is due to its property of initiating the process of oxidation, thanks to the particular stabilisation of the triplet activated states.

In Photochem. Photobiol., 50(3), 359 (1989) it has been seen that four impurities originate from ketoprofen under aerobic conditions, (3-benzoylphenyl) ethane, (3-benzoylphenyl) ethyl hydroperoxide, (3-benzoylphenyl) ethanol and (3-benzoylphenyl) ethanone, whilst, under aerobic conditions, only 3-benzoylphenyl ethane is formed.

In WO9520387 was highlighted the importance of selecting the appropriate moment for the application of active substances, which in the case of photosensitive substances was limited to nocturnal periods.

In WO9205769 has been described a composition containing a UV filter and S(+) ketoprofen with the aim of preventing or curing the solar erythema by UV radiation arising from exposure to the same. The ketoprofen undergoes photodegradation reactions and the addition of a UV filter alone does not seem capable, under the conditions and in the concentrations used, to eliminate this problem; for which the compositions described do not seem satisfactory.

JP60155111 describes pharmaceutical formulations for external use, containing ketoprofen, in concentrations of from 0.1 to 10%, together with a W filter, in concentrations of from 0.01 to 5%, selected from the group of derivatives of p-amino benzoic acid, anthranilic acid, benzophenone, salicylic acid or aminoacids and, if necessary, an antioxidant, in concentrations of from 0.01 to 5%, selected from the group of ascorbic acid, tocopherol methyl p-hydroxybenzoate, butyl hydroxy toluene, butyl hydroxy anisole. In example 1 it is shown that, in gels containing 2-hydroxy-4-methoxy benzophenone (benzofenone-3), as a UV filter, in concentrations of from 0% al 1%, the percentage of ketoprofen not broken down following an exposure of 8 hours to solar light is maximal and equal to 90% of the initial value with concentrations of UV filter equal to 1%. In example 3 it is noted that, in gel type formulations, amongst the various UV filters taken into consideration at 1%, the best protection was given by benzophenone-3 (with a residue of non degraded ketoprofen equal to 87%) whilst the other UV filters have a worse result (e.g. 2-hydroxy-4-methoxybenzophenone-5-sulphonic acid, otherwise known as benzophenone-4 or sulisobenzone, reaches a value of 80%). Still with example 3 it is noted that the association of benzophenone-3 at 1% with, as an antioxidant, tocopherol at 1% reaches a value of non degraded ketoprofen equal to 95%. From examination of the JP60155111 document, and in particular from the examples described in it, those skilled in the art can expeditiously deduce that for the realisation of formulations containing ketoprofen, and in particular for gels, whereby avoiding the phenomenon of photodegradation, is required:
the presence of a UV filter at a maximum concentration of 1 % and amongst the UV filters benzofenone-3 is absolutely preferable
the possible additional presence of an antioxidant in variable percentages according to the antioxidant itself, *i.e*. tocopherol 1 - 3%, methyl p-hydroxy benzoate 0,2%, dibutyl hydroxy toluene 0.01 - 0.5%, butyl hydroxy anisole 0.02%.

All that has been described however does not permit avoidance of all the problems connected with the photodegradation of ketoprofen.

In FR2804024 the authors describe an invention on topical pharmaceutical forms containing non steroidal anti-inflammatory agents (NSAls), in particular ketoprofen, protected against photodegradation by solar filters selected from the group:
derivatives of cinnamic acid, dibenzoyl methane, benzophenone, p-amino benzoic acid with the possible presence of an antioxidant selected from: butyl hydroxy anisole, butyl-p-cresole, palmityl ascorbate, or tocopherol.

In FR2804024 are taken into particular consideration UV filters which protect against UV B rays (claim 2).

The percentage of UV filter claimed varies from 0.5 to 10% whilst the range of possible use of the optional antioxidant is not cited.

By observing the specific examples described it is deduced that the UV filters preferred have been: an ethoxylate derivative of p-amino benzoic acid at concentrations of 4 or 2% (examples 1-9), ethyl p-methoxy cinnamate at a concentration of 3% (example 10) and sulisobenzone at a concentration of 4% (example 11), all in combination with butyl hydroxy anisole as antioxidant at a concentration of 0.05%.

The solutions presented, and in particular the examples described, in FR2804024 have not however shown themselves to be completely satisfactory in the case of ketoprofen.

In fact to solve the problems connected with the protection of ketoprofen from photodegradation, it is not sufficient to take into consideration its protection from UV rays, but it is necessary to also consider other problems:
the UV rays which it is necessary to filter are predominantly UV A and not UV B
it is necessary to use UV filters which do not bestow, due to their nature, sensitisation problems to the skin
it is necessary to take account of the penetration of the active ingredient into the dermis, which must not be impeded or slowed down by the filter selected the final formulation must meet with the 'compliance ' of the patient.

It is known from the literature (Gonzalez *et al.* Journal of the Medical Academy of Dermatology 1996, 35: 871-885), that the action spectrum at the basis of many photodermatoses is located within the UV A range (320-400 nm) and therefore preventive photoprotection based exclusively on the use of UV B filters has limited therapeutic benefit. The filters derived from p-amino benzoic acid (used for example in examples 1-9 of FR2804042) and octyl methoxy cinnamate (used in example 10 of FR2804042) are examples of filters selective for UV-B, but not for UV-A rays.

Furthermore, it is remembered that UV A radiation is implicated in the majority of photoallergenic reactions, in the delayed hypersensitivity response, as also in some phototoxicity reactions and idiopathic dermatoses.

Above all, it must be underlined that UV A rays penetrate deeper into the cuteous, reaching the dermis, in difference to UV B rays, which do not pass through the epidermis.

It being known that the NSAls, in particular ketoprofen, penetrate deeply, reaching the dermis (and also the underlying tissues, e.g. joints), and remaining at such a level for various days, it is intuitive that it is useful to block UV A rays with the help of a suitable filter in order that these do not photodegrade the ketoprofen, giving rise to the known phenomena of photoallergenicity/phototoxicity.

Within the family of the benzophenonic filters, it is known from the literature (Alanko K. Contact Dermatitis 2001, 44: 188 - Durieu C. Ann. Dermatol. Venereol. 2001; 128:1020-1024), that benzophenone-3 (greatly preferred as the UV filter in JP60155111) has demonstrated a cross sensitisation (implicit within the molecule itself) and a cross photosensitisation (therefore consequent to its photodegradation products), with ketoprofen. It therefore seems evident that the use of benzophenone-3, as a UV A filter in formulations for topical use containing ketoprofen, could even exacerbate the allergic and photoallergenic phenomena associated with ketoprofen itself.

In relation to the antioxidants used in the preparations for topical use cited in FR2804024 and JP60155111, are employed substances such as ascorbic acid, tocopherol, methyl p-hydroxy benzoate, butyl hydroxy toluene (BHT), butyl hydroxy anisole (BHA).

These, together with other molecules, such as propyl gallate, are commonly used in the formulation of pharmaceutical preparations for their antioxidant activities.

From the data in the literature (Bosca F. *et*. *al*. Journal of Photochemistry and Photobiology 1995; 31:133-138 - Duval C. and M.C. Poelan Journal of Pharmaceutical Sciences 1995; 84:107-110) it emerges that butyl hydroxy anisole (BHA) is more effective in its antioxidant activities with respect to various derivatives of tocopherol (acetate, succinate, linoleate) and even reduced glutathione, drastically reducing the lipoperoxidation of linoleic acid generated by the photodegradation of ketoprofen.

In relation to the use of derivatives of p-hydroxybenzoic acid (such as methyl p-hydroxy benzoate, an antimicrobial preserving agent) it is known from the literature (American Pharmaceutical Association Handbook of Pharmaceutical Excipients 3^{rd} edition 2000:342) that such compounds can give rise to allergic reactions when applied topically and therefore their use is not advisable in dermatological products.

In relation to the use of ascorbic acid and its derivatives in aqueous solutions, it is known that they oxidise rapidly losing their activity. Water being one of the most common solvents used for the preparation of creams and gels for topical application, these antioxidants are therefore not indicated for such use.

With reference to BHT, it is indicated that this molecule, at the concentrations useful for guaranteeing adequate antioxidant activity, is particularly irritating to the cuteous (Michael and Irene Ash Handbook of Pharmaceutical Additives 1997:360 - American Pharmaceutical Association Handbook of Pharmaceutical Excipients 3^{rd} edition 2000:51-52 - Roed-Peterson J. Br. J. Dermatol. 1976; 94:233-241- Juhlin L. Br. J. Dermatol. 1981; 104:369-381).

Finally, concerning the use of propyl gallate, as an antioxidant in formulations for topical use, it is known from the literature (Cronin E. Contact Dermatitis Edinburgh: Churchill Livingstone 1980: 170-177 - Brun R. Dermatologica 1970; 140:390 - Pigatto PD Contact Dermatitis. 1984 Jul; 11:43) that the derivatives of gallic acid are potentially highly allergenic and can cause hyperkeratosic eczema developing allergic dermatitis by contact.

Finally concerning the problem of 'compliance', from the topical formulations already present on the market containing ketoprofen, we can draw from the teachings that a transparent gel, which is not greasy like creams, which can be easilly applied without particular massaging and which is invisible, is particularly appreciated by patients.

In JP60155111 it is taught that, in particular for gels, it is necessary to use a UV filter selected from a sufficiently large group of compounds, in concentrations from 0.01 to 5% in association, if necessary, with an antioxidant, selected from the representative group, at concentrations between 0.01 and 5%, to resolve the problems connected with the photodegradation of ketoprofen: in reality this assertion is not correct and is, on the basis of the results obtained also misleading to the expert in the art.

In fact in example 3 of JP60155111 various gels containing ketoprofen and a UV filter at a concentration of 1% are compared: these filters are not all these declared, but only cinoxate, 2,2'-dihydroxy-4-methoxy-benzophenone (benzophenone-8), methyl anthranilate, 2-hydroxy-4-methoxy-benzophenone (benzophenone-3), the 2-ethylhexylic ester of 4-dimethylamino-benzoic (acid), benzophenone-4, phenyl salicylate, and benzophenone-3 + tocopherol at a concentration of 1%; it arises that the percentage of protection offered by the UV filters is very variable making them absolutely unequal to each other. Of preference according to the data shown are without a doubt benzophenone -8 and benzophenone -3. The addition of tocopherol as an antioxidant further improves the protection offered by benzophenone-3.

In example 1 is shown how the protection offered by various concentrations of benzophenone-3 in gels with ketoprofen varies; the concentrations are of 0.1 0.2 0.5 and 1.0 % benzofenone-3. It appears clear that only the concentration of 1% manages to sufficiently avoid the photodegradation of ketoprofen. The same is repeated, with analogous results, in example 4.

The use of anioxidants is envisaged, in case of necessity; and these are selected from a group consisting, but only some are cited, of in particular tocophereol, methyl p-hydroxy benzoate, dibutyl hydroxy toluene and butyl hydroxy anisole. In the examples, the antioxidant, when used, is present in various concentrations according to the antioxidant itself. For the gels only tocopherol at a concentration of 1% is reported together with benzophenone -3 at a concentration of 1% as a UV filter (example 11) whilst other oxidants are used for other types of formulations, such as the emulsions or creams; in particular butyl hydroxy anisole appears in the composition of an oily cream (example 28) at a concentration of 0.02% with benzophenone -3 at 1% as a UV filter.

Therefore, from a careful examination of the tests carried out and of the examples described in JP60155111, it could be concluded that, to realise pharmaceutical compositions, in particular gels, containing ketoprofen, benzofenone-3 will be preferable as a UV filter at a concentration of 1%, and possibly, as an antioxidant, tocopherol at 1%; these compositions would seem able to eliminate the photodegradation of the ketoprofen, but on the basis of the existing literature they would seem to suffer from other problems connected with the choice of benzophenone -3 itself.

In FR2804024, a good 11 examples of pharmaceutical formulations have been described; in particular:
Examples from 1 to 9: these formulations contain ethoxylate esters of p-amino benzoic-acid (UV filter) and butyl hydroxy anisole (antioxidant) at 0.05%, but we have been able to note that the decomposition of ketoprofen was still present following irradiation (up to 35% of the initial quantity), furthermore problems of phototoxicity of the formulations were however present and the permeability across human skin was considerably reduced if compared with known formulations containing ketoprofen without UV filters (such as Ketum);
in example 10 the use of octyl methoxy cinnamate together with butyl hydroxy anisole does not prevent the photodegradation of tenoxicam and problems relating to permeability across the skin caused by the large quantity of surfactant used can be easily hypothesised;

The formulation envisaged in example 11 with sulisobenzone at a concentration of 4% and butyl hydroxy anisole at 0,05% and with the anti-inflammatory agent dictophenac at 2.5% which is not of any interest to us; but a totally similar formulation using ketoprofen instead of diclophenac, has been assayed and has emerged as being phototoxic (see Table 1 Example 7).

The examples from 1 to 10 used specific filters for UV B, and therefore are not suitable for correct photoprotection, and that described in example 11 does not seem to be transferable for use with ketoprofen.

In addition all the above examined compositions, when placed in comparison with analogous transparent gels, but without UV filters, already on the market, were not satisfactory with reference to their physico-chemical characteristics (coloration, lack of viscosity, change of odour etc.) and possible problems of 'compliance' with the patients under treatment could easily be hypothesised.

In conclusion all that is described in the prior art and in particular in JP60155111 and FR280424, could also instruct to an expert in the art how to increase the photostability of pharmaceutical formulations containing ketoprofen, but not, until now, in a completely satisfactory manner, and however not how to prevent the problems of phototoxicity, of photoallergenicity and reduced permeability across the skin and a quicker analgesic effect, problems which still remain to be correctly resolved.

### Description of the invention

Surprisingly it has been found that only formulations containing ketoprofen, or its S(+) ketoprofen isomer, together with benzophenone-4 (or sulisobenzone) as UV filter, butyl hydroxy anisole (BHA) at dosages comprised of between 0.05% and 0.2%, as an antioxidant, and other excipients, such as fragrances, substances for facilitating cutaneous absorption, solvents and other pharmaceutically acceptable excipients, show, next to a marked reduction in the photodegradation of ketoprofen (Table 5), an optimal prevention of the phototoxic/photoallergenic characteristics (Tables 3 and 4) together with adequate *in vitro* permeation (Tables 6 and 7).

It should be noted that the concentration of the antioxidant agent is extremely important and absolutely not predictable on the basis of the prior art, in fact, as highlighted in Table 1, at concentrations of butyl hydroxy anisole < 0.05 % the formulation has emerged as being phototoxic *in vitro.*

In the case of use, as UV filters, of some benzophenonic derivatives, of anthranilic acid, salicylic acid, p-amino benzoic acid (such as PEG-25 PABA), of compounds based on aminoacids as described in JP60155111, in FR2804024, or of derivatives of cinnamic acid (octyl methoxy cinnamate), a photoprotection, even if in general only partial, of the active ingredient (ketoprofen) was observed, but the system emerged however as being phototoxic and the physico-chemical properties were significantly changed; also the addition of an antioxidant, according to that explicitly envisaged by JP60155111 and FR2804024, did not succeed in completely resolving the above mentioned problems.

A pharmaceutical formulation containing ketoprofen and BHA at adequate doses was assayed for its phototoxicity (see TAB. 2 with formulation Ex. 8) and it was shown to be inadequate.

In the case of benzophenone-4 (sulisobenzone), as a UV filter, the active ingredient (ketoprofen) emerged as being stable and a phototoxicity potential comparably much lower with respect to other UV filters was observed. This reduced phototoxicity, could surprisingly be further reduced only with an adequate quantity of BHA. Other antioxidant substances such as alpha tocopherol, BHT, or ascorbic acid, did not show effects comparable to adequate doses of BHA.

In addition, also the problems of permeability across the cuteous, shown by the formulations already described containing ketoprofen and/or its isomers, together with a UV filter and possibly an antioxidant agent, could be resolved.

Therefore, the present invention describes pharmaceutical formulations containing ketoprofen or its isomers in high percentages, but having negligible phototoxic and photoallergenic effects, a high permeability in the *in vitro* tests, an optimal conservation of the pharmacological quality of the active ingredient itself (ketoprofen), with reduced formation of photodegradation impurities, with respect to other known formulations still based on ketoprofen, without UV filters, such as, for example, Ketum gel.

In the case of spray gel formulations under pressure, immediately after application an analgesic effect surprisingly high, higher even in respect of the same placebo of Fastum gel spray, was noted; probably a synergetic effect takes place between the analgesic action of ketoprofen and the cooling action due to the presence of the propelling gas under pressure.

The topical foundations, as the basis of the present invention, are characterised thus:
0.5 - 5% by weight of ketoprofen or S(+) ketoprofen or a mixture of the two isomers
1 - 5% by weight of benzophenone-4 (sulisobenzone) as a UV filter
BHA, as an antioxidant, in concentrations greater than 0,05 and up to 0,2% by weight
and, the residual content, up tp 100% by weight, of pharmaceutically acceptable excipients such as co-solvents, fragrances, stimulants for absorption, etc.

The active ingredient contained in the above mentioned pharmaceutical preparations is selected from ketoprofen, as a racemic mixture, the S(+) ketoprofen isomer or mixtures of the two isomers of ketoprofen; these are to be considered in both the free acid form or in the form of pharmaceutically acceptable salts selected from the group of the salts of sodium, potassium, calcium, magnesium, tromethamine, hydroxy ethylamine, di(hydroxyethyl)-amine, tri(hydroxyethyl)-amine, lysine, arginine, or in mixed form consist of free acid and its salt selected from the group above.

The percentage of active ingredient, calculated as free acid in the racemic form, contained in the formulation, can vary from 1% to 5% by weight (calculated from the total weight of the formulation), and in particular between 2% and 5% by weight; particularly preferred are the ratios between 2.5% and 3% by weight.

When only the S(+) isomer is considered, the percentage of active ingredient, calculated as free acid, can vary between 0.5% and 2.5% by weight and in particular between 1% and 2.5% by weight; particularly preferred are the ratios between 1.25% and 1.5% by weight.

The percentage of UV filter is comprised of between 1% and 5% by weight, preferably between 2% and 4% by weight. The percentage of antioxidant is greater than 0.05% and up to 0.2% by weight, preferably comprised of between 0.075% and 0.15% by weight.

The formulations according to the present invention, can contain in addition, as pharmaceutically acceptable excipients, adjuvants such as water or monoalcohols such as ethanol, vitamins amongst which tocopherol, dexpanthenol, retinol palmitate, thickeners, protective colloids, humectants, fragrances, amongst which the preferred is lavender oil, polyoils, electrolytes, gelifiers, agents for increasing the permeability across the cuteous, polymers or copolymers, emulsifiers, emulsion stabilisers, etc.

As preservants the substances with low allergenic power such as ethyl alcohol, benzylic alcohol are preferred.

As substances which facilitate absorption across the cuteous liposomes, ethyl alcohol, diethylene glycol monoethyl ether, medium chain triglycerides EP, urea, dimethyl sulphoxide (DMSO) are preferred.

Amongst the gelifiers, particularly suited, are selected from the group: carbomers, in particular carbomer 940, polyacrylamide isoparaffin laureth-7, xanthan gum, carrageenin, acacia gum, guar gum, agar gel, alginates and methyl hydroxy cellulose, carboxy methyl cellulose, hydroxy propyl cellulose, hydroxy propyl methylcellulose, hydroxyethyl cellulose, ethyl cellulose, polyacrylates, polyvinyl alcohol, polyvinylpyrrolidone, colloidal silica, etc.

Amongst the hydrating agents urea and panthenol can be considered.

According to the excipients used, the topical formulations subject of the invention can be therefore in the form of gels, gel sprays, lotions, non greasy creams.

In the case of spray gel form under pressure suitable propellers are those chosen among: compressed nitrogen and trifluoroethane 134a; particularly preferred is the trifluoroethane 134a. On the contrary propellers like alkanes of low molecular weight, as propane or butane or their mixture, are not suitable.

The pharmaceutical formulations subject of the present invention can be realised by mixing the components of the same, according to the methods well known to the experts in the art:

Following herein is reported as an example, the general formula of a topical pharmaceutical formulation, realised according to the present invention:
2.5 - 3% ketoprofen
2 - 4% (UV filter) benzophenone-4 (sulisobenzone)
0.075 - 0.15% (antioxidant) BHA
0-20% permeability promoters
0 - 0.5% fragrance
20 - 50% ethanol
sufficient purified water 100%

The pharmaceutical formulations described can be used in the topical treatment of inflammatory pathologies or skeletal muscle infirmities such as, for example, myalgia, myositis, sprains, contusions.

Following herein, some non limiting examples, of topical pharmaceutical, formulations, realised according to the present invention. All the ingredients are indicated as percentages by weight, unless specified otherwise.

### Examples.

### Example 1

The formulation has been carried out by mixing the single ingredients according to the techniques known in the state, of the art,

| | |
|---|---|
| ketoprofen | 2.5 |
| carbomer 940 | 1.4 |
| hydroxy ethyl cellulose | 0.5 |
| ethyl alcohol 96° | 32 |
| lavender oil | 0.1 |
| benzophenone-4 | 3 |
| triethanolamine | 4 |
| BHA | 0,1 |
| Sufficient purified water | 100 |

Preparation: dissolve part of the triethanolamine in all the purified water necessary, then add the benzophenone-4 and mix untill dissolved. Separately prepare the alcoholic solution of ketoprofen, BHA, lavender. Add this solution to the aqueous solution of benzophenone-4 and mix until a clear solution is achieved. Add the carbomer 940 and homogenise until the complete reswelling of the polymer. Adjust the pH with triethanolamine and homogenise.

Add the hydroxyethyl cellulose and mix to complete homogenisation.

### Example 2

| | |
|---|---|
| ketoprofen | 2.5 |
| carbomer 940 | 1.3 |
| ethyl alcohol 96° | 45 |
| lavender oil | 0.1 |
| benzophenone-4 | 3 |
| triethanolamine | 4 |
| BHA | 0.1 |
| Sufficient purified water | 100 |

### Sufficient propellant

Preparation: dissolve part of the triethanolamine an all the purified water necessary, then add the benzophenone-4 and mix till dissolved. Separately prepare the alcoholic solution of ketoprofen, BHA, lavender. Add this solution to the aqueous solution of benzophenone-4 and mix until achieving a clear solution. Add the carbomer 940 and homogenise until the complete reswelling of the polymer. Adjust the pH with triethanolamine and homogenise. Under pressure distribute into canisters with a suitable propellant (compressed nitrogen or tetrafluoroethane 134a).

### Example 3

| | |
|---|---|
| ketoprofen | 2.5 |
| polyacrylamide isoparaffin laureth-7 | 2 |
| ethyl alcohol 96° | 32 |
| lavender oil | 0.1 |
| benzophenone-4 | 2 |
| triethanolamine (sufficient) | pH 5.9-6.5 |
| BHA | 0.1 |
| Sufficient purified water | 100 |

Preparation: dissolve part of the triethanolamine in all the purified water necessary, then add the benzophenone-4 and mix until dissolved. Separately prepare the alcoholic solution of ketoprofen, BHA, lavender. Add this solution to the aqueous solution of benzophenone-4 and mix until achieving a clear solution. Add the polyacrylamide isoparaffin laureth-7 and homogenise until the complete reswelling of the polymer. Adjust the final pH with the necessary triethanolamine and homogenise.

### Example 4

| | |
|---|---|
| ketoprofen | 2.5 |
| ethyl alcohol 96° | 40 |
| lavender oil | 0:1 |
| benzophenone-4 | 3 |
| BHA | 0.1 |
| Medium chain triglycerides EP (sufficient) | 100 |

Preparation: dissolve in the ethyl alcohol the ketoprofen, the benzophenone-4, lavender oil, BHA. Add the triglycerides and mix till homogenised.

### Example 5

| | |
|---|---|
| ketoprofen | 2.5 |
| carbomer 940 | 1.5 |
| ethyl alcohol 96° | 32 |
| diethylene glycol monoethyl ether | 15 |
| lavender oil | 0.1 |
| benzophenone-4 | 3 |
| BHA | 0.1 |
| triethanolamine (sufficient) | 5.9-6.5 |
| Sufficient purified water | 100 |

Preparation: dissolve part of the triethanolamine ain all the purified water necessary, then add the benzophenone-4 and mix until dissolved. Separately prepare the alcoholic solution of ketoprofen, BHA, lavender oil. Add the diethylene glycol monoethyl ether and mix until obtaining a clear solution. Add this solution to the aqueous solution of benzophenone-4 and mixed to homogeneity. Adjust the final pH with the necessary triethanolamine and homogenise.

### Example 6

| | |
|---|---|
| ketoprofen | 2.5 |
| anhydrous colloidal silica | 1 |
| ethyl alcohol 96° | 40 |
| lavender oil | 0.1 |
| benzophenone-4 | 3 |
| BHA | 0.1 |
| Medium chain triglycerides EP (sufficient) | 100 |

Preparation: dissolve in the ethyl alcohol the ketoprofen, benzophenone-4, lavender oil, BHA. Add the triglycerides and mix to homogeneity. Add the colloidal silica and homogenise to complete dispersion.

### Example 7 (COMPARISON)

The formulation has been carried out by mixing the single ingredients according to the techniques known in the state of the art

| | |
|---|---|
| ketoprofen | 2.5 |
| carbomer 940 | 1.8 |
| ethyl alcohol 96° | 32 |
| lavender oil | 0.1 |
| benzophenone-4 | 4 |
| triethanolamine | (sufficient) |
| BHA | 0.05 |
| Sufficient purified water | 100 |

### Example 8 (COMPARISON)

The formulation has been carried out by mixing the single ingredients according to the techniques known in the state of the art

| | |
|---|---|
| ketoprofen | 2.5 |
| ethyl cellulose N22 | 0.5 |
| ethyl alcohol 96° | 50 |
| lavender oil | 0.1 |
| octyl methoxy cinnamate | 2 |
| BHA | 0.1 |
| Medium chain triglycerides EP (sufficient) | 100 |

### DESCRIPTION OF THE TESTS ASSAY OF PHOTOTOXICITY IN BALB/C 3T3 CELLS (INCORPORATION OF NEUTRAL RED)

The study has been conducted in accordance with the GLP guidelines of the U.S.F.D.A. (21CRFPart.58, 22nd December 1978) and later revisions; according to the Community Directive 1999/11/EC of 8th March 1999 (adoption by the "OECD Principles on Good Laboratory Practices as revised in 1997") and later revisions and with Legislative Decree 27th January 1992, N°120 and later revisions.

In addition the study has been conceived to comply with the experimental methods of the Guidelines: EEC Council Directive 2000/33, Annex II, B.41.

The assay in subject is directed towards the *in vitro* analysis of the potential phototoxicity of a substance under test through the measurement of the incorporation of the dye neutral red.

The results obtained, in Tables 1, 2, 4, 6, are related to the toxicity induced by the substance under test, the ketoprofen, following irradiation with UVA and are reported as percentages of the values for the negative controls, wherein for negative control is meant the cell cultures treated with the solvent used. The evaluation of the data is carried out through the calculation of the photoirritation factor (PIF). The PIF is determined thus: PIF = EC50 (-UV)/EC50 (+UV) *i.e.* it is the concentration of the substance which reduces by 50% the absorbance (read with a spectrofotomer at 530 nm) of the cell culture when not exposed to UV radiation [EC50 (-UV)] compared to when exposed to UV radiation [EC50 (+UV)]. It must be considered that a PIF value less than or greater than 5 distinguishes the non phototoxic from the phototoxic agents. In the case in which the substance is cytotoxic only following irradiation, then the resulting ">PIF" is calculated which is: ">PIF" = Cmax (-UV)/ EC50 (+UV). Only ">PIF" values with values >1 are predictive of potential phototoxins.

This cut-off value has been defined over the course of the validation process of the test as an alternative to the *in vivo* test.

The validity of the test is verified through treatment with a positive control substance (8-methoxypsoralene), well characterised for its efficacy in this experimental system, both in our laboratories, and through results available in the literature.

The tests have been carried out by comparing the commercial formulations Fastum® gel and Ketum gel (carbomer based hydroalcoholic gels) with the relevant Placebo formulation and the formulation of example 7 (with benzophehone-4 and BHA 0.05%) and the relevant Placebo.

For Placebo is meant the complele formulation without the ketoprofen active ingredient.

**Table 1**

| Formulation | EC₅₀mean +UV [mg/ml] | EC₅₀mean -UV [mg/ml] | PIF | >PIF |
|---|---|---|---|---|
| Placebo Fastum® gel | NC | NC | NC | NC |
| Fastum® gel | 0.15 | NC | NC | 65.05 |
| Placebo Ketum gel | NC | NC | NC | NC |
| Ketum gel | 0.12 | NC | NC | 89.68 |
| Ketoprofen UV gel Placebo (Example 7) | NC | NC | NC | NC |
| Ketoprofen UV gel (Example 7) | 0.44 | NC | NC | 57.62 |

| | | | | |
|---|---|---|---|---|
| EC= cytotoxic concentration | | | | |
| NC = not calculable | | | | |

Conclusions: preparations containing ketoprofen, benzophenone-4 and BHA at a concentration of 0.05% (as in example 7) have demonstrated a potential phototoxicity in the *in vitro* test, showing analogous phototoxicity to that for the commercial formulations without UV filters (Fastum and Ketum) and the absence of phototoxicity for the respective placebos (therefore NC).

**Table 2 KETOPROFENE LOTION with octyl methoxy cinnamate (example 8) Cellular phototoxicity test (Neutral Red Uptake)**

| Formulation | EC₅₀av.+UV [µg/mL] | EC₅₀av.-UV [µg/mL] | PIF | >PIF |
|---|---|---|---|---|
| Ket. Lotion with OMC (Ex.8) | 87,3 | NC | NC | 1,15 |

| | | | | |
|---|---|---|---|---|
| NC = not calculable | | | | |

Conclusions: from our direct experience, the use of octyl methoxy cinnamate in preparations for topical use containing ketoprofen and butyl hydroxy anisole as antioxidant, have not shown optimal protection in the *in vitro* phototoxicity tests.

In fact, regarding the Ketoprofen gel spray formulation with octyl methoxy cinnamate, it is possible to calculate the EC50 value after having irradiated with UVA. In this case it has been therefore possible to calculate the ">PIF" which has emerged greater than 1 therefore indicating a potential phototoxicity of the substance under test".

**Table 3 KETOPROFEN GEL with benzophenone-4 (example 1) Cellular phototoxicity test (Neutral Red Uptake)**

| Formulation | EC₅₀av.+UV [µg/mL] | EC₅₀av.-UV [µg/mL] | PIF | >PIF |
|---|---|---|---|---|
| Ket. Gel with benz.4(Ex 1) | NC | NC | NC | NC |

| | | | | |
|---|---|---|---|---|
| NC = not calculable | | | | |

**Table 4 KETOPROFEN SPRAY with benzophenone-4 (example 2) Cellular phototoxicity test (Neutral Red Uptake)**

| Formulation | EC₅₀av.+UV [µg/mL] | EC₅₀av.-UV [µg/mL] | PIF | >PIF |
|---|---|---|---|---|
| Ket. Spray gel with benz.4(Ex.2) | NC | NC | NC | NC |

| | | | | |
|---|---|---|---|---|
| NC = not calculable | | | | |

Conclusions: preparations, both in gel form (Example 1) and in gel spray form (Example 2), containing ketoprofen, benzophenone 4, and BHA at a concentration of 0,1% have not demonstrated potential phototoxicity in the *in vitro* test, confirming the importance of the quantity of antioxidant with the aim of reducing the adverse events consequent to the production of free radicals.

### IN VITRO PHOTODEGRADATION ASSAY

With the aim of verifying the efficacy of the benzophenone - 4 solar filter in the protection of ketoprofen from photodegradation, a photostability study has been carried out on a series of products based on ketoprofen and containing the filters in subject in conformity with the guidelines "ICH TOPIC Q1 B - Photostability testing of new Active Substances and Medicinal Products (CPMP/ICH/279/95) in comparison to a Ketum gel in current production (lot. 2001/160).

### Operating conditions

The test has been carried out on the following products:
Ketoprofen UV (gel) - Example 1.
Ketoprofen UV (spray) - Example 2

Four aliquots of around 1 g of each lot of gel have been irradiated for 1 hour under the following conditions of illumination:
179 Klux within the visible range and 79 W/m² in the UV region.

At time 0 and following 10', 20', 30', 60' minutes of exposure to light the following analyses have been carried out on the gel samples: titration and impurities (by HPLC)

In the products containing the benzophenohe-4 filter, a good photoprotection with respect to the Ketum gel product is observed, as highlighted by a much restrained diminution in the ketoprofen titre and by a much reduced formation of impurities.

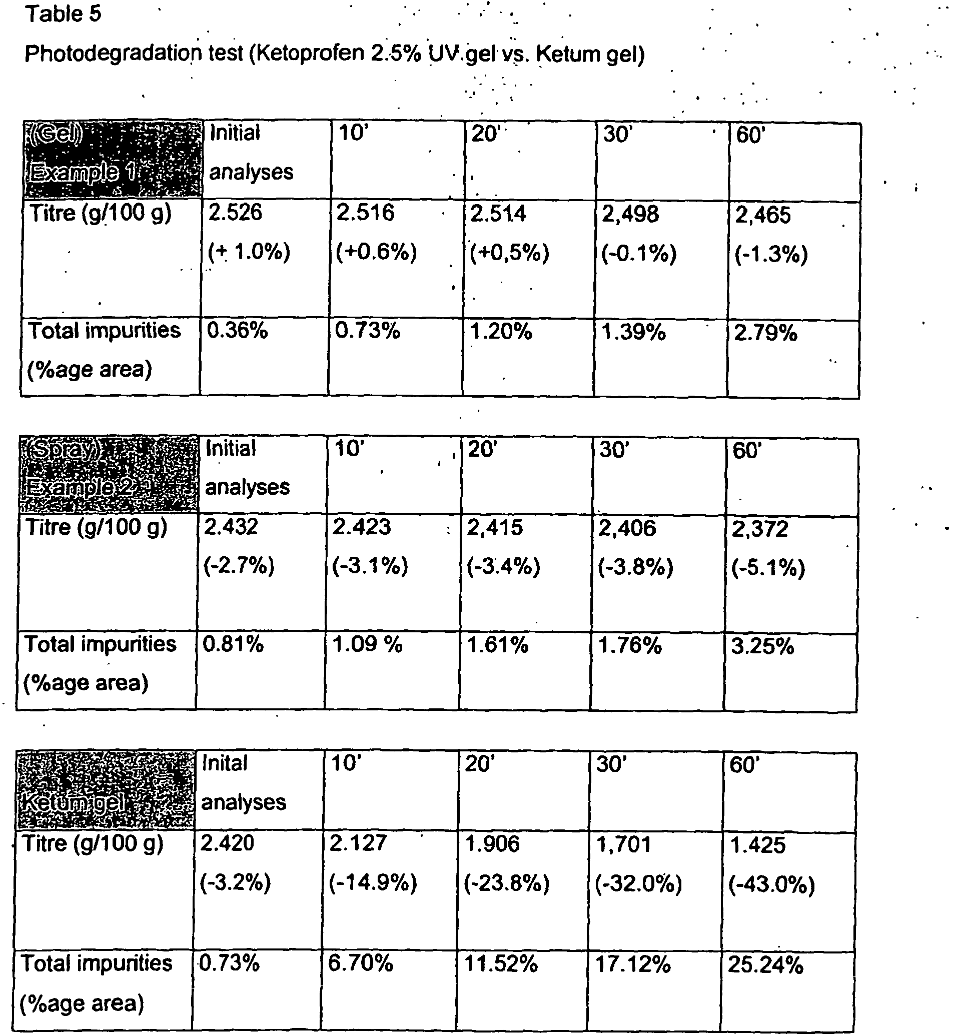

### Conclusions

The filter used (Benzophenone - 4) seems to possess good efficacy as a photoprotector towards ketoprofen in both the formulations analysed.

### IN VITRO PERMEABILITY ASSAY (SYNTHETIC MEMBRANE)

The *in vitro* diffusion study has been carried out using an apparatus consist of 6 Chien Cells. Such a cell being constituted of a horizontal system of two compartments between which is interposed a membrane (in the case under test a nitrate/cellulose acetate synthetic membrane).

The two compartments act, respectively the upper and lower, as Donor and Receptor.

Inside the Donor is placed the substance, the diffusion of which it is intended to evaluate, inside the Receptor, is a suitable medium to simulate the *in vivo* compartment. The Receptor compartment is thermostatically set to 35°C using a circulating bath; the Donor compartment is maintained in contact with the environment. The flow of active ingredient across the membrane with time is determined by removing at preset time intervals (0, 1, 2, 3, 4, 5, 6, 7, 8 hours) an aliquot of Receptor medium, which is then replenished, so as to maintain the volume of Receptor medium constant. The sample volume thus removed is subjected to analysis for the determination of the active ingredient content (ketoprofen).

The analytical control has been performed through (UV) spectroscopic methods. All the activities for conducting the test have been carried out according to Good Laboratory Practice (GLP).

The apparatus which has been used for the present study is constituted of 6 cells allowing the execution of 3 tests for the substance under test and for the reference substance per session.

**Table 6**

| Sample time | Fastum | Ketum | Example 1 |
|---|---|---|---|
| (hours) | (mg/cell) | (mg/cell) | (mg/cell) |
| 1 | 0.93 | 0.77 | 0.91 |
| 2 | 1.51 | 1,52 | 1.51 |
| 3 | 1.7 | 1,71 | 1.68 |
| 4 | 1.72 | 1,71 | 1.68 |
| 5 | 1.58 | 1,58 | 1.61 |
| 6 | 1.52 | 1,48 | 1.42 |
| 8 | 1.55 | 1,52 | 1.44 |

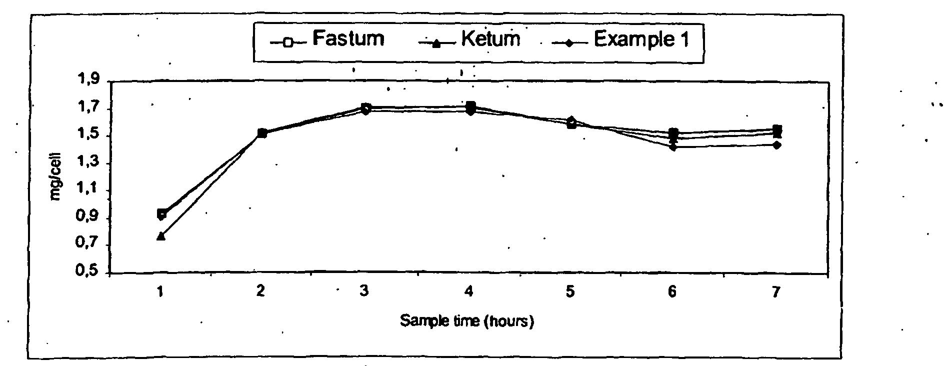

**Table 7**

| Sample time | Fastum | Ketum | Example 2 |
|---|---|---|---|
| (hours) | (mg/cell) | (mg/cell) | (mg/cell) |
| 1 | 1.02 | 0.98 | 0.74 |
| 2 | 1.62 | 1.48 | 1.34 |
| 3 | 1.66 | 1.62 | 1.64 |
| 4 | 1.71 | 1.60 | 1.70 |
| 5 | 1.68 | 1.48 | 1.61 |
| 6 | 1.55 | 1.38 | 1.51 |

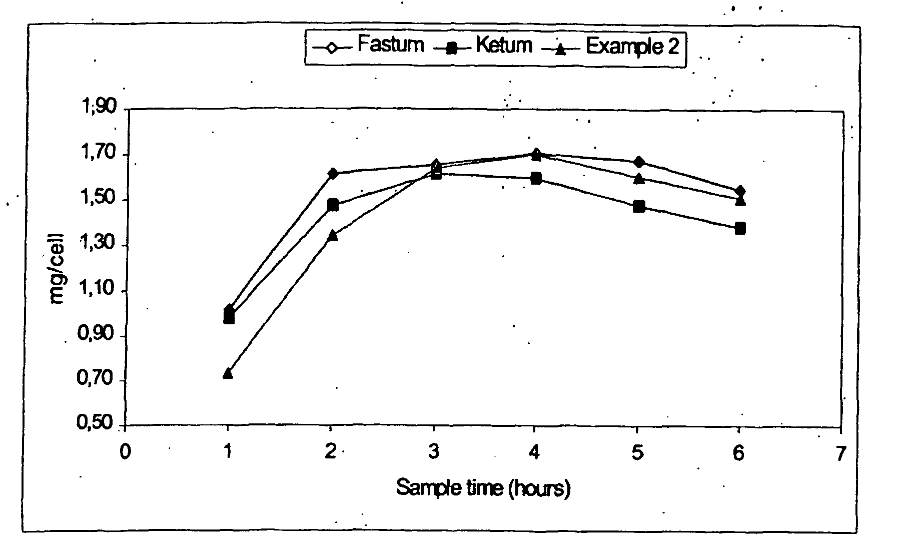

Conclusions: the formulations (Example 1 and Example 2) containing ketoprofen, benzophenone-4, BHA (0.1%), demonstrate the possession of, thanks to the adequate ratio between co-solvents and polymers, superimposable *in vitro* permeation characteristics to these of the commercial products of reference (Fastum® gel, Ketum gel).

### ANALGESIC ASSAY

In order to verify the analgesic activity of the formulation of example 2 an assay on animals (rats) was performed.

20 male rats (weight 170 - 220 g.) were studied. A modified Randall and Selitto (Arch. Int. Pharmacodyn. 1957, 111:409-19) method was used to measure the pain threshold. The pain index (P.I) was calculated according to Tsukada et al. (Arzneim. Forsch. Drug Res. 1978, 28: 428-38) (Pain Index = pain threshold after injection of the inflammatory agent/pain threshold before injection of the inflammatory agent).

0,1 ml of a suspension 1% karragenin are injected subplantary in the posterior paw of the animals.

After two hours a defined quantity (70 mg/rat) of the tested formulations are applied topically on the injected paw and the injection point is massaged delicately

for a given time in order to assure an uniform distribution and penetration of the active principle through the skin.

The formulation in the form of gel was weighted before the application white for the spray formulation the supplying time releasing the same quantity in weight of the gel was measured.

An immediate evaluation of the pain index was performed (P.I: imm.) after the application of the products.

The pain Index was evaluated 2 hours after the application of the products (P.I. 2 hours).

In the assay Fastum gel, its placebo, the gel spray (composition of example 2) and its placebo were compared.

The results are reported in Table 8.

| Medicam. | Doses (mg/rat) | N° animals | P.I. (imm) | P.I. (2 hrs) |
|---|---|---|---|---|
| Placebo | 70 | 5 | 0.555 | 0.561 |
| Fastum gel - | 70 | 5 | 0.548 | 1.073 |
| Placebo | 70 | 5 | 0.823 | 0.466 |
| Spray gel (Ex.2) | 70 | 5 | 1.160 | 1.285 |

Conclusions: the spray gel formulation (according to Ex. 2) containing ketoprofen, benzophenone4, BHA, and a suitable propeller (trifluoroethane 134a, which allows the production of aerosol cans under pressure) shows surprising immediate analgesic properties whcih after 2 hrs are higher than the gel product. Such properties are surprisingly higher even in respect of the same placebo of the spray gel immediately after application. It is supposed that a synergetic effect take place between the analgesic action of ketoprofen and the cooling action due to the supply of the propelling gas under pressure.

## Claims

1. A topical pharmaceutical composition **characterised in that** it comprises:
a) ketoprofen in the form of free acid or its pharmaceutically acceptable salts or mixtures thereof;
b) sulisobenzone;
c) butyl hydroxy anisole
possibly in combination with pharmaceutically acceptable excipients wherein the butyl hydroxy anisole is present in concentrations comprised between 0,05% - 0,2% by weight.

2. The composition according to claims 1 in which the ketoprofen consists of a mixture of its two isomers or their pharmaceutically acceptable salts.

3. The composition according to claims 1 in which the ketoprofen consists of a racemic mixture of its two isomers or their pharmaceutically acceptable salts.

4. The composition according to claims 1 in which the ketoprofen consists of the S(+) isomer or its pharmaceutically acceptable salts.

5. The composition according to claims 1 - 4 in which the ketoprofen consists of a mixture formed from the acid form and by the respective salified form.

6. The pharmaceutical composition according to claims 1-5, in which the percentage of ketoprofen, calculated as free acid, as a mixture of isomers varying from 0.5 to 5% by weight, or, as S (+) isomer, from 0.5 to 2.5% by weight.

7. The pharmaceutical composition according to claim 6, in which the percentage of ketoprofen, calculated as free acid, as a mixture of isomers varying from 2 to 5% by weight, or, as the S (+) isomer, from 1 to 2.5% by weight.

8. The pharmaceutical composition according to claim 7, wherein the percentage of ketoprofen, calculated as free acid, as a mixture of isomers varying from 2.5 to 3% by weight, or, as the S (+) isomer, from 1.25 to 1.5% by weight.

9. The topical pharmaceutical composition according to claims 1 - 8, wherein the percentage of sulisobenzone as UV filter is comprised of from 2 to 4%.

10. The pharmaceutical composition according to claims 1-9 wherein the percentage of butyl hydroxy anisole as antioxidant agent varies between 0.075 and 0.15 % by weight.

11. The pharmaceutical composition according to claims 1-10 **characterised by** the general formulation: 2.5 - 3% ketoprofen, 2-4% benzophenone-4 (sulisobenzone), 0.075 - 0.15% (antioxidant) BHA, 0 - 20% permeability promoters, 0 - 0,5% fragrances, 20 - 50% ethanol, sufficient purified water 100%.

12. The composition according to claims 1 - 11 wherein the pharmaceutically acceptable salts of ketoprofen are selected from the group consist of salts of sodium, potassium, calcium, magnesium, tromethamine, hydroxy ethylamine, di(hydroxyethyl)-amine, tri(hydroxyethyl)-amine, lysine, arginine.

13. The formulations according to claims 1-12 wherein the pharmaceutically acceptable excipients are selected from the group consist of : adjuvants, vitamins, thickeners, protective colloids, humectants, fragrances, polyoils, electrolytes, gelifiers, polymers or copolymers, emulsifiers, emulsion stabilisers, preservants, liposomes, ethyl alcohol, diethylene glycol monoethyl ether, medium chain triglycerides EP, urea, dimethyl sulphoxide (DMSO), carbomer, isoparaffin laureth-7, xanthan gum, carrageenin, acacia gum, guar gum, agar gel, alginates and methyl hydroxy cellulose, carboxymethylcellulose, hydroxypropyl cellulose, hydroxypropyl methyl cellulose, hydroxyethyl cellulose, ethyl cellulose, polyacrylates, polyvinyl alcohol, polyvinylpyrrolidone, colloidal silica, panthenol.

14. Pharmaceutical composition according to claims 1 - 13 in the form of gel, spray gel lotion, non oily cream suitable for the topical treatment of inflammatory pathologies or skeletal muscle infirmities selected from: myalgias/myositis, sprains, contusions.

15. Pharmaceutical compositions according to claim 14 containing ketoprofen in the form of spray gel **characterised by** the presence of a propeller under pressure.

16. Pharmaceutical composition according to claim 15 wherein the propeller is chosen in the group consisting of: nitrogen under pressure and tetrafluoroethane134a.

17. Pharmaceutical compositions according to claim 16 wherein the propeller is tetrafluoroethane134a.

18. Pharmaceutical composition according to claims 14-17 comprising:
a) ketoprofen in racemic form or as a mixture of its isomers or as isomer (+) in the form of free acid or as pharmaceutically acceptable salts thereof or their mixture, in a concentration, calculated as free acid, of 0.5 - 5% by weight;
b) sulisobenzone, as UV filter, in a concentration of 2-4% by weight
c) butyl hydroxyl anisole, as anti-oxidant, in a concentration of 0.075-0.15% by weight;
possibly in combination with pharmaceutically acceptable additives.

19. Pharmaceutical compositions according to claims 1 to 18 in the form of gels, spray gels, lotions, non greasy creams suitable for the topical treatment of inflammatory pathologies or skeletal muscle infirmities selected from: myalgias/myositis, sprains, contusions.

20. The use of sulisobenzone in percentages from 2 to 4%, as a UV filter, for the preparation of pharmaceutical compositions containing ketoprofen according to claim 1 and 14, for the topical treatment of inflammatory pathologies.

21. The use of butyl hydroxy anisole in percentages from 0.075 to 0.15%, as an antioxidant agent, for the preparation of pharmaceutical compositions containing ketoprofen according to claim 1 and 14, for the topical treatment of inflammatory pathologies.

## Patentansprüche

1. Topische pharmazeutische Zusammensetzung, **dadurch gekennzeichnet, dass** sie umfasst:
a) Ketoprofen in Form der freien Säure oder seiner pharmazeutisch zulässigen Salze oder Mischungen davon;
b) Sulisobenzon;
c) Butylhydroxyanisol
gegebenenfalls zusammen mit pharmazeutisch zulässigen Hilfsstoffen, wobei Butylhydroxyanisol in Konzentrationen zwischen 0,05 Gew.-% - 0,2 Gew.-% vorhanden ist.

2. Zusammensetzung gemäß Anspruch 1, in welcher Ketoprofen aus einer Mischung von zwei seiner Isomere oder seiner pharmazeutisch zulässigen Salze besteht.

3. Zusammensetzung gemäß Anspruch 1, in welcher Ketoprofen aus einer racemischen Mischung von zwei seiner Isomere oder seiner pharmazeutisch zulässigen Salze besteht.

4. Zusammensetzung gemäß Anspruch 1, in welcher Ketoprofen aus dem S(+)-Isomer oder seinem pharmazeutisch zulässigen Salz besteht.

5. Zusammensetzung gemäß einem der Ansprüche 1 - 4, in welcher Ketoprofen aus einer Mischung besteht, die aus der Säureform und der entsprechenden versalzten Form gebildet wird.

6. Pharmazeutische Zusammensetzung gemäß einem der Ansprüche 1 - 5, in welcher der Prozentsatz an Ketoprofen, berechnet als freie Säure, als eine Isomerenmischung zwischen 0,5 und 5 Gew.-%, oder, als S (+)-Isomer, zwischen 0,5 und 2,5 Gew.-% variiert.

7. Pharmazeutische Zusammensetzung gemäß Anspruch 6, in welcher der Prozentsatz an Ketoprofen, berechnet als freie Säure, als eine Isomerenmischung zwischen 2 und 5 Gew.-%, oder, als S (+)-isomer, zwischen 1 und 2,5 Gew.-% variiert.

8. Pharmazeutische Zusammensetzung gemäß Anspruch 7, in welcher der Prozentsatz an Ketoprofen, berechnet als freie Säure, als eine Isomerenmischung zwischen 2,5 und 3 Gew.-%, oder, als S (+)-Isomer, zwischen 1,25 und 1,5 Gew.-% variiert.

9. Topische pharmazeutische Zusammensetzung gemäß einem der Ansprüche 1 - 8, in welcher der Prozentsatz an Sulisobenzon als UV-Filter zwischen 2 und 4 Gew.-% beträgt.

10. Pharmazeutische Zusammensetzung gemäß einem der Ansprüche 1 - 9, in welcher der Prozentsatz an Butylhydroxyanisol als Antioxidationsmittel zwischen 0,075 und 0,15 Gew.-% variiert.

11. Pharmazeutische Zusammensetzung gemäß einem der Ansprüche 1 - 10, **gekennzeichnet durch** die allgemeine Formulierung: 2,5 - 3 % Ketoprofen, 2 - 4 % 4-Benzophenon (Sulisobenzon), 0,075 - 0,15 % (Antioxidationsmittel) BHA, 0 - 20 % Permeabilitätsförderer, 0 - 0,5 % Duftstoffe, 20 - 50 % Ethanol, ausreichend gereinigtes Wasser 100 %.

12. Zusammensetzung gemäß einem der Ansprüche 1 - 11, in welcher die pharmazeutisch zulässigen Salze von Ketoprofen ausgewählt wird aus der Gruppe bestehend aus Salzen mit Natrium, Kalium, Calcium, Magnesium, Tromethamin, Hydroxyethylamin, Di(hydroxyethyl)amin, Tri(hydroxyethyl)amin, Lysin, Arginin.

13. Formulierungen gemäß einem der Ansprüche 1 - 12, in welcher die pharmazeutisch zulässigen Hilfsstoffe ausgewählt werden aus der Gruppe bestehend aus: Zusatzstoffen, Vitaminen, Verdickungsmitteln, Schutzkolloide, Feuchthaltemitteln, Duftstoffen, Polyölen, Elektrolyten, Gelbildungsmitteln, Polymeren oder Copolymeren, Emulgatoren, Emulsionsstabilisierungsmittel, Konservierungsmittel, Liposomen, Ethylalkohol, Diethylenglykol, Monoethylethern, mittelkettigen Triglyceriden EP, Harnstoff, Dimethylsulfoxid (DMSO), Carbomer, Isoparaffin Laureth-7, Xanthangummi, Carrageenan, Gummi arabicum, Guar, Agar-Agar, Alginaten und Methylhydroxycellulose, Carboxymethylcellulose, Hydroxypropylcellulose, Hydroxypropylmethylcellulose, Hydroxyethylcellulose, Ethylcellulose, Polyacrylaten, Polyvinylalkohol, Polyvinylpyrrolidon, kolloidalem Siliciumdioxid, Panthenol.

14. Pharmazeutische Zusammensetzung gemäß einem der Ansprüche 1 - 13 in Form eines Gels, einer Sprühgellotion, einer nicht öligen Creme, die für die topische Behandlung von entzündlichen Krankheiten oder Skelettmuskelerkrankungen geeignet sind, ausgewählt aus: Myalgien/Myositis, Verstauchungen, Prellungen.

15. Pharmazeutische Zusammensetzung gemäß Anspruch 14, umfassend Ketoprofen in Form eines Sprühgels, **gekennzeichnet durch** die Anwesenheit eines unter Druck stehenden Treibmittels.

16. Pharmazeutische Zusammensetzungen gemäß Anspruch 15 in welchem das Treibmittel ausgewählt wird aus der Gruppe bestehend aus: unter Druck stehendem Stickstoff und Tetrafluorethan 134a.

17. Pharmazeutische Zusammensetzungen gemäß Anspruch 16, in welchen das Treibmittel Tetrafluorethan 134a ist.

18. Pharmazeutische Zusammensetzung gemäß einem der Ansprüche 14 - 17, umfassend:
a) Ketoprofen in racemischer Form oder als Mischung seiner Isomere oder als (+)-Isomer in Form der freien Säure oder als pharmazeutisch zulässiges Salz davon oder deren Mischungen, in einer Konzentration, berechnet als freie Säure, von 0,5 - 5 Gew.%;
b) Sulisobenzon, als UV-Filter, in einer Konzentration von 2 - 4 Gew.%;
c) Butylhydroxyanisol, als Antioxidationsmittel, in einer Konzentration von 0,075 - 0,15 Gew.%;
gegebenenfalls zusammen mit pharmazeutisch zulässigen Zusatzstoffen.

19. Pharmazeutische Zusammensetzungen gemäß einem der Ansprüche 1 - 18 in Form von Gels, Sprühgellotionen, nicht öligen Cremes, die für die topische Behandlung von entzündlichen Krankheiten oder Skelettmuskelerkrankungen geeignet sind, ausgewählt aus: Myalgien/Myositis, Verstauchungen, Prellungen.

20. Verwendung von Sulisobenzon in Prozentangaben von 2 bis 4 %, als ein UV-Filter, für die Herstellung von Ketoprofen enthaltenden pharmazeutischen Zusammensetzungen nach Anspruch 1 und 14, für die topische Behandlung von entzündlichen Erkrankungen.

21. Verwendung von Butylhydroxyanisol in Prozentangaben von 0,075 bis 0,15 %, als Antioxidationsmittel, für die Herstellung von Ketoprofen enthaltenden pharmazeutischen Zusammensetzungen nach Anspruch 1 und 14, für die topische Behandlung von entzündlichen Erkrankungen.

## Revendications

1. Composition pharmaceutique topique **caractérisée en ce qu'**elle comprend :
- (a) du kétoprofène sous forme acide libre ou sous forme de ses sels pharmaceutiquement acceptables ou de mélanges de ceux-ci ;
- (b) de la sulisobenzone :
- (c) du butylhydroxyanisole
éventuellement en combinaison avec des excipients pharmaceutiquement acceptables, et dans laquelle le butylhydroxyanisole est présent à des concentrations comprises entre 0,05 et 0,2 % en poids.

2. Composition selon la revendication 1, dans laquelle le kétoprofène consiste en un mélange de ses deux isomères ou de leurs sels pharmaceutiquement acceptables.

3. Composition selon la revendication 1, dans laquelle le kétoprofene consiste en un mélange racémique de ses deux isomères ou de leurs sels pharmaceutiquement acceptables.

4. Composition selon la revendication 1, dans laquelle le kétoprofène consiste en l'isomère S(+) ou en ses sels pharmaceutiquement acceptables.

5. Composition selon les revendications 1 à 4, dans laquelle le kétoprofène consiste en un mélange formé à partir de la forme acide et par la forme salifiée respective.

6. Composition pharmaceutique selon les revendications 1 à 5, dans laquelle le pourcentage de kétoprofène calculé en acide libre, sous la forme d'un mélange d'isomères, varie de 0,5 à 5 % en poids, ou sous la forme de l'isomère S (+), varie de 0,5 à 2,5 % en poids.

7. Composition pharmaceutique selon la revendication 6, dans laquelle le pourcentage de kétoprofène calculé en acide libre, sous la forme d'un mélange d'isomères, varie de 2 à 5 % en poids, ou sous la forme de l'isomère S (+), varie de 1 à 2,5 % en poids.

8. Composition pharmaceutique selon la revendication 7, dans laquelle le pourcentage de kétoprofène calculé en acide libre, sous la forme d'un mélange d'isomères, varie de 2,5 à 3 % en poids, ou sous la forme de l'isomère S (+), varie de 1,25 à 1,5 % en poids.

9. Composition pharmaceutique topique selon les revendications 1 à 8, dans laquelle le pourcentage de la sulisobenzone utilisée en tant que filtre UV, est compris entre 2 et 4 %.

10. Composition pharmaceutique selon les revendications 1 à 9, dans laquelle le pourcentage du butylhydroxyanisole utilisé en tant qu'agent anti-oxydant, varie de 0,075 à 0,15 % en poids.

11. Composition pharmaceutique selon les revendications 1 à 10, **caractérisée par** le formulation générale : 2,5 à 3 % de kétoprofène, 2 à 4 % de benzophénone-4 (sulisobenzone), 0,075 à 0,15 % de BHA (anti-oxydant), 0 à 20 % de promoteurs de perméabilité, 0 à 0,5 % de fragrances, 20 à 50 % d'éthanol, eau purifiée en quantité suffisante pour compléter à 100 %.

12. Composition selon les revendications 1 à 11, dans laquelle les sels pharmaceutiquement acceptables de kétoprofène sont choisis au sein du groupe consistant en des sels de sodium, de potassium, de calcium, de magnésium, de trométhamine, d'hydroxyéthylamine, de di(hydroxyéthyl)amine, de tri(hydroxyéthyl)amine, de lysine, d'arginine.

13. Formulations selon les revendications 1 à 12, dans lesquelles les excipients pharmaceutiquement acceptables sont choisis au sein du groupe consistant en des adjuvants, vitamines, épaississants, colloïdes protecteurs, humectants, fragrances, polyols, électrolytes, gélifiants, polymères ou copolymères, émulsifiants, stabilisateurs d'émulsion, conservateurs, liposomes, alcool éthylique, monoéthyléther de diéthylèneglycol, triglycérides EP à chaîne médiane, urée, diméthylsulfoxyde (DMSO), carbomer, lauréth-7 d'isoparaffine, gomme xanthane, carraghénine, gomme acacia, gomme de guar, gel d'agar, alginates et méthylhydroxycellulose, carboxyméthylcellulose, hydroxypropylcellulose, hydroxypropylméthylcellulose, hydroxyéthylcellulose, éthylcellulose, polyacrylates, polyvinylalcool, polyvinylpyrrolidone, silice colloïdale, panthénol.

14. Composition pharmaceutique selon les revendications 1 à 13, sous forme de gel, de gel spray, de lotion, de crème non huileuse, appropriée au traitement topique de pathologies inflammatoires ou d'infirmités de muscles squelettique choisies parmi les myalgies/myosites, entorses, contusions.

15. Compositions pharmaceutiques selon la revendication 14, contenant du kétoprofène sous forme de gel spray, **caractérisées par** la présence d'un propulseur sous pression.

16. Composition pharmaceutique selon la revendication 15, dans laquelle le propulseur est choisi au sein du groupe consistant en de l'azote sous pression et du tétrafluoroéthane 134a.

17. Compositions pharmaceutiques selon la revendication 16, dans laquelle le propulseur est le tétrafluoroéthane 134a.

18. Composition pharmaceutique selon les revendications 14 à 17, comprenant :
- (a) du kétoprofène sous la forme racémique ou sous la forme d'un mélange de ses isomères ou sous la forme d'isomère (+) sous forme d'acide libre ou sous la forme de ses sels pharmaceutiquement acceptables ou de leurs mélanges, à une concentration calculée en acide libre, de 0,5 à 5 % en poids ;
- (b) de la sulisobenzone en tant que filtre UV, à une concentration de 2 à 4 % en poids :
- (c) du butylhydroxyanisole en tant qu'anti-oxydant, à une concentration de 0,075 à 0,15 % en poids ;
éventuellement en combinaison avec des additifs pharmaceutiquement acceptables.

19. Compositions pharmaceutiques selon les revendications 1 à 18, sous forme de gels, de spray gels, de lotions, de crèmes non graisseuses, appropriées au traitement topique de pathologies inflammatoires ou d'infirmités de muscles squelettique choisies parmi les myalgies/myosites, les entorses et contusions.

20. Utilisation de la sulisobenzone à des pourcentages de 2 à 4 % en tant que filtre UV pour la préparation de compositions pharmaceutiques contenant du kétoprofène selon les revendications 1 et 14, pour le traitement topique de pathologies inflammatoires.

21. Utilisation du butylhydroxyanisole à des pourcentages de 0,075 à 0,15 % en tant qu'agent anti-oxydant pour la préparation de compositions pharmaceutiques contenant du kétoprofène selon les revendications 1 et 14, pour le traitement topique de pathologies inflammatoires.
